# EUROPEAN PATENT APPLICATION

(11) **EP 3 909 558 A1**
(43) Date of publication of application: **17.11.2021**
(21) Application number: 20752646.8
(22) Date of filing: 07.02.2020
(51) Int. Cl.: A61H 1/02, A61B 5/11, A61B 5/00, A63B 23/16, A63B 24/00, A63B 21/00

(54) **HAND REHABILITATION EXERCISE DEVICE**

(30) Priority: 08.02.2019 KR 20190014972; 28.08.2019 KR 20190105695
(71) Applicant: NEOFECT Co., Ltd., Seongnam-si, Gyeonggi-do 13449 (KR)
(72) Inventor: PARK, Byung Geol, Icheon-si, Gyeonggi-do 17383 (KR); CHOI, Young Geun, Yongin-si, Gyeonggi-do 16990 (KR); YOO, Kyung Hwan, Ganghwa-gun, Incheon 23051 (KR); ROH, Sung Jun, Seoul 06335 (KR); KIM, Hyun Soo, Seoul 06354 (KR); JEON, Joon Ha, Seoul 08831 (KR)
(74) Representative: Kurig, Thomas
(86) International application number: PCT/KR2020/001737
(87) International publication number: WO 2020/162698

(57) **Abstract**

The present disclosure relates to a hand rehabilitation exercise device, including: a finger movement sensing sensor disposed on a finger and senses a movement of the finger; a back of hand worn unit communicatively connected to the finger movement sensing sensor in a wired or wireless manner and worn on the back of a hand; and a finger worn unit having a finger movement sensing sensor attaching/detaching unit to/from which the finger movement sensing sensor is attached/detached, and which is worn on the finger, wherein the finger worn unit is worn on a specific finger joint, the back of hand worn unit is worn on the back of a hand, and then the finger movement sensing sensor is mounted on the finger movement sensing sensor attaching/detaching unit.

## Description

### TECHNICAL FIELD

The present disclosure relates to a hand rehabilitation exercise device.

### BACKGROUND ART

In the case of a stroke or Parkinson's disease, various physical changes appear, depending on states the diseases have reached. In particular, these diseases are commonly accompanied by phenomena in which hands are paralyzed and fingers are contracted.

If the paralysis of the hands and the contraction of the fingers are just left as they are continually, muscles or joints may harden, and the patient may feel pain when moving and may have difficulty in normal activities even though nerves are restored.

Also, in addition to the case of the specific diseases described above, there are many cases in which the movement of fingers is impaired due to an unexpected accident.

Therefore, in such a case, it is very important to maintain the exercise ability as much as possible by promoting blood circulation and neural communication by performing rehabilitation treatment to continuously move the paralyzed or disabled hand.

A related art invention for such a hand rehabilitation exercise device is disclosed in Korean Patent No. 10-1541082.

As disclosed, the conventional hand rehabilitation exercise device is configured to dispose a finger sensing unit for sensing movement of each finger joint of a rehabilitation exerciser, for example, a first joint of a finger located adjacent to a palm and a second joint adjacent to a free end of the finger, wherein each finger sensing unit is fixed by enclosing the corresponding joint of the finger with a fixing string.

In addition, the conventional hand rehabilitation exercise device is configured to dispose a first connection unit in the form of a flat cable connecting a back of hand sensing unit disposed on a back of hand and a first joint sensing unit disposed on the first finger joint of the rehabilitation exerciser as a finger sensing unit along the length direction of fingers.

However, in this conventional hand rehabilitation exercise device, in the case of a rehabilitation exerciser in a state in which the fingers are contracted, the first connection part (cable) does not move in the movement direction of the contracted fingers when the rehabilitation exerciser performs joint motion of stretching and clenching fingers, but moves along the length direction of fingers. Hence, friction occurs between the finger and the cable during joint exercise of the finger, so that the movement of the finger is not smooth and the movement of the finger is disturbed.

In addition, since the finger sensing unit of the conventional hand rehabilitation exercise device is integrally formed without being separated from the string-type finger fixing unit for fixing the finger sensing unit to the finger, most rehabilitation exercisers were a hand rehabilitation exercise device used in a state in which the fingers are contracted, and thus it is difficult to wear the device in a state in which the fingers are spread out.

In addition, since the conventional finger exercise device has a predetermined interval between a back of hand worn unit and a finger sensing unit, there is a drawback in that various finger exercise devices have to be manufactured to correspond to various finger lengths.

In addition, the conventional finger exercise device has a drawback in that it is inconvenient to wear because it is necessary to first wear the back of hand worn unit on a back of hand and then fix the finger sensing unit to each finger using the finger fixing unit.

### DISCLOSURE OF INVENTION

### TECHNICAL PROBLEM

An aspect of the present disclosure is directed to providing a hand rehabilitation exercise device that can easily be worn to improve user convenience, and can be worn by replacing only a cable in correspondence with various finger lengths, so that the device can be used regardless of the length of fingers.

In addition, another aspect of the present disclosure is directed to providing a hand rehabilitation exercise device having an improved wearing sensation by disposing a control unit mounted on a printed circuit board on a wrist, and which enables a rehabilitation exerciser to perform rehabilitation exercises without feeling discomfort when the rehabilitation exerciser moves the back of his or her hand.

In addition, another aspect of the present disclosure is directed to providing a hand rehabilitation exercise device capable of reducing friction between a cable and a finger during rehabilitation exercise and enabling smooth rehabilitation exercises without interfering with the movement of the finger.

In addition, another aspect of the present disclosure is directed to providing a hand rehabilitation exercise device that can be easily worn even in a state in which the fingers are contracted, thereby enabling a rehabilitation exercise.

### SOLUTION TO PROBLEM

An aspect of the present disclosure is directed to providing a hand rehabilitation exercise device, including: a finger movement sensing sensor disposed on a finger and senses a movement of the finger; a back of hand worn unit communicatively connected to the finger movement sensing sensor in a wired or wireless manner and worn on the back of a hand; and a finger worn unit having a finger movement sensing sensor attaching/detaching unit to/from which the finger movement sensing sensor is attached/detached, and which is worn on the finger, wherein the finger worn unit is worn on a specific finger joint, the back of hand worn unit is worn on the back of a hand, and then the finger movement sensing sensor is mounted on the finger movement sensing sensor attaching/detaching unit.

In addition, in another embodiment, an aspect of the present disclosure is directed to providing a hand rehabilitation exercise device, including: a finger movement sensing sensor disposed on a finger and sensing a movement of the finger; a wrist worn unit communicatively connected to the finger movement sensing sensor in a wired or wireless manner and worn on a wrist; and a finger worn unit having a finger movement sensing sensor attaching/detaching unit to/from which the finger movement sensing sensor is attached/detached, and which is worn on the finger, wherein the finger worn unit is worn on a specific finger joint, the wrist worn unit is worn on a wrist, and then the finger movement sensing sensor is mounted on the finger movement sensing sensor attaching/detaching unit.

As an embodiment, the finger worn unit may include a finger tube coupled to a finger joint; and a finger movement sensing sensor attaching/detaching unit provided on one side of the finger tube to/from which the finger movement sensing sensor is attached/detached.

As another embodiment, the finger worn unit includes a pair of mounting rings which forms a finger mounting hole on which a finger is mounted and is disposed at a distance from each other; and a finger movement sensing sensor attaching/detaching unit connected to the pair of mounting rings and to/from which the finger movement sensing sensor is attached/detached.

The finger movement sensing sensor and the back of hand worn unit are electrically connected by a cable extending in a length direction of the finger, and the cable may be curvedly stretched and contracted according to joint motion of a finger worn on the finger worn unit.

The hand rehabilitation exercise device may further include a back of hand movement sensing sensor disposed on the back of hand worn unit and senses a movement of a back of hand.

The hand rehabilitation exercise device may further include a control unit provided in the back of hand worn unit to receive and collect each piece of movement data of a finger and a back of hand sensed by the finger movement sensing sensor and the back of hand movement sensing sensor.

The finger movement sensing sensor and the wrist worn unit are electrically connected by a cable extending in a length direction of the finger, and the cable may be curvedly stretched and contracted according to joint motion of a finger worn on the finger worn unit.

The hand rehabilitation exercise device may further include a wrist movement sensing sensor disposed on the wrist worn unit and sensing a movement of a wrist.

The hand rehabilitation exercise device may further include a control unit provided in the wrist worn unit to receive and collect each piece of movement data of a finger and a wrist sensed by the finger movement sensing sensor and the wrist movement sensing sensor.

The hand rehabilitation exercise device may further include a wrist worn unit worn on a wrist; and a wrist movement sensing sensor disposed on the wrist worn unit and sensing a movement of a wrist.

The back of hand worn unit and the wrist worn unit may be communicatively connected in a wired or wireless manner.

The hand rehabilitation exercise device may further include a control unit provided in the back of hand worn unit or the wrist worn unit to receive and collect each piece of movement data of a finger, back of hand, and wrist sensed by the finger movement sensing sensor, the back of hand movement sensing sensor, and the wrist movement sensing sensor.

In another embodiment, an aspect of the present disclosure is directed to providing a hand rehabilitation exercise device, including: a plurality of finger movement sensing sensors which are disposed on each of a plurality of fingers and sense a movement of each finger; a plurality of cables respectively connected to the plurality of finger movement sensing sensors and extended by a length toward a wrist; a back of hand worn unit that forms a path for the plurality of cables and is worn on the back of a hand; a wrist worn unit worn on the wrist to which the plurality of cables are connected; and a control unit mounted on a printed circuit board and provided in the wrist worn unit to receive and collect finger movement data sensed by the plurality of finger movement sensing sensors through each of the plurality of cables.

The device further includes a back of hand movement sensing sensor provided in the back of hand worn unit to sense a movement of a back of hand; and an auxiliary cable electrically connecting the control unit and the back of hand movement sensing sensor, wherein the control unit may receive and collect back of hand movement data sensed by the back of hand movement sensing sensor through the auxiliary cable.

The back of hand worn unit may further include a back of hand movement sensing sensor attaching/detaching unit to/from which the back of hand movement sensing sensor is attached/detached.

The hand rehabilitation exercise device may further include a cable aligning unit provided in the back of hand worn unit, and fixes or movably aligns the plurality of cables.

The cable aligning unit may be disposed on a back of hand adjacent to the thumb, and the back of hand movement sensing sensor attaching/detaching unit is disposed at a central area of the back of hand.

The hand rehabilitation exercise device may further a wrist movement sensing sensor provided in the wrist worn unit and senses a movement of a wrist, wherein the control unit may receive and collect wrist movement data sensed by the wrist movement sensing sensor.

The hand rehabilitation exercise device may further include a finger worn unit having a finger movement sensing sensor attaching/detaching unit to/from which the finger movement sensing sensor is attached/detached, and worn on the finger.

The plurality of cables may be made of a bendable circular cross-section wire, and may have a length corresponding to joint motion of each finger according to a state of each finger.

The hand rehabilitation exercise device may further include a pressure measuring sensor provided in the finger worn unit worn on the thumb, and measures pressure generated on a finger in which any one of an index finger, middle finger, ring finger, and pinky finger is in contact, wherein the control unit may receive and collect pressure data measured by the pressure measuring sensor.

### ADVANTAGEOUS EFFECTS OF INVENTION

According to the present disclosure, the hand rehabilitation exercise device can be easily worn to improve user convenience, and can be worn by replacing only a cable in correspondence with various finger lengths, so that the device can be used regardless of the length of fingers.

In addition, the hand rehabilitation exercise device can improve the wearing sensation by disposing a control unit mounted on a printed circuit board on a wrist, and enables a rehabilitation exerciser to perform rehabilitation exercises without feeling discomfort when the rehabilitation exerciser moves his/her back of the hand.

In addition, the hand rehabilitation exercise device can reduce friction between a cable and a finger during rehabilitation exercise and enable a smooth rehabilitation exercises without interfering with the movement of the finger.

In addition, the hand rehabilitation exercise device can be easily worn even in a state in which the fingers are contracted, thereby enabling a rehabilitation exercise.

### BRIEF DESCRIPTION OF DRAWINGS

FIG. 1 is a perspective view of a hand rehabilitation exercise device according to a first embodiment of the present disclosure.
FIG. 2 is an exploded perspective view of the main part of FIG. 1.
FIG. 3 is a rear perspective view of FIG. 1.
FIG. 4 is a view illustrating a state in which the hand rehabilitation exercise device of FIG. 1 is worn on the hand.
FIG. 5 is a view illustrating a state of joint motion of fingers using the hand rehabilitation exercise device of FIG. 1.
FIG. 6 is a perspective view of a hand rehabilitation exercise device according to a second embodiment of the present disclosure.
FIG. 7 is a rear perspective view of FIG. 6.
FIG. 8 is a view illustrating a state in which the hand rehabilitation exercise device of FIG. 6 is worn on the hand.
FIG. 9 is a view illustrating a state of joint motion of fingers using the hand rehabilitation exercise device of FIG. 6.
FIG. 10 is a front view of a hand rehabilitation exercise device according to a third embodiment of the present disclosure.
FIG. 11 a rear view of FIG. 10.
FIG. 12 is a front view of a state in which the hand rehabilitation exercise device according to the third embodiment of the present disclosure is worn.
FIG. 13 is a rear view of a state in which the hand rehabilitation exercise device according to the third embodiment of the present disclosure is worn.
FIG. 14 is a view illustrating a hand rehabilitation exercise in a state in which the hand rehabilitation exercise device according to the third embodiment of the present disclosure is worn.
FIG. 15 is a view illustrating a state of pinching the thumb and index finger in a state in which the hand rehabilitation exercise device according to the third embodiment of the present disclosure is worn.
FIG. 16 is a cross-sectional view taken along line A-A' of FIG. 10.

### DESCRIPTION OF EMBODIMENTS

The advantages and features of the present disclosure and methods of achieving them will be apparent from the embodiments that will be described in detail with reference to the accompanying drawings. It should be noted, however, that the present disclosure is not limited to the following embodiments, and may be implemented in various different forms. Rather the embodiments are provided so that this disclosure will be thorough and complete and will fully convey the scope of the present disclosure to those skilled in the technical field to which the present disclosure pertains.

Terms used in the specification are used to describe embodiments of the present disclosure and are not intended to limit the scope of the present disclosure. In the specification, the terms of a singular form may include plural forms unless otherwise specified. The expressions "comprise" and/or "comprising" used herein indicate existence of one or more other elements other than stated elements but do not exclude presence of additional elements. Like reference denotations refer to like elements throughout the specification. As used herein, the term "and/or" includes each and all combinations of one or more of the mentioned elements. It will be understood that, although the terms "first", "second", etc., may be used herein to describe various elements, these elements should not be limited by these terms. These terms are only used to distinguish one element from another element. Accordingly, a first element mentioned below could be termed a second element without departing from the technical ideas of the present disclosure.

Unless otherwise defined, all terms (including technical and scientific terms) used herein have the same meaning as commonly understood by those skilled in the technical field to which the present disclosure pertains. It will be further understood that terms, such as those defined in commonly used dictionaries, should not be interpreted in an idealized or overly formal sense unless expressly so defined herein.

Hereinafter, embodiments will be described in detail with reference to the accompanying drawings.

Prior to the description, in various embodiments, the same reference numerals are used for the constituents having the same configuration and illustrated in the first exemplary embodiment, and in other embodiments, configurations different from the first embodiment will be described.

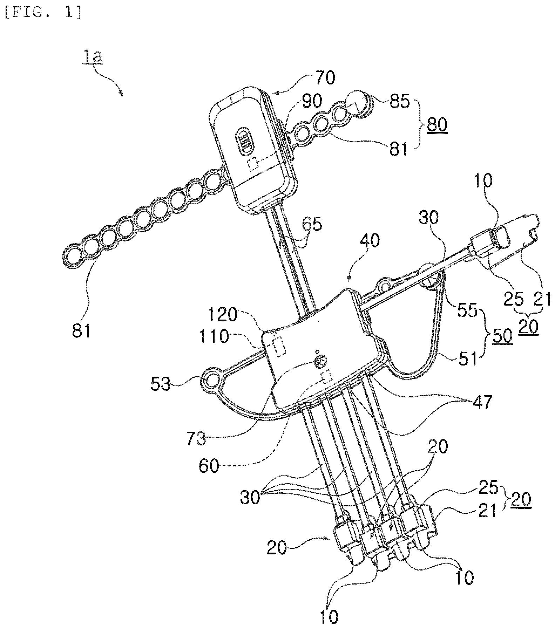

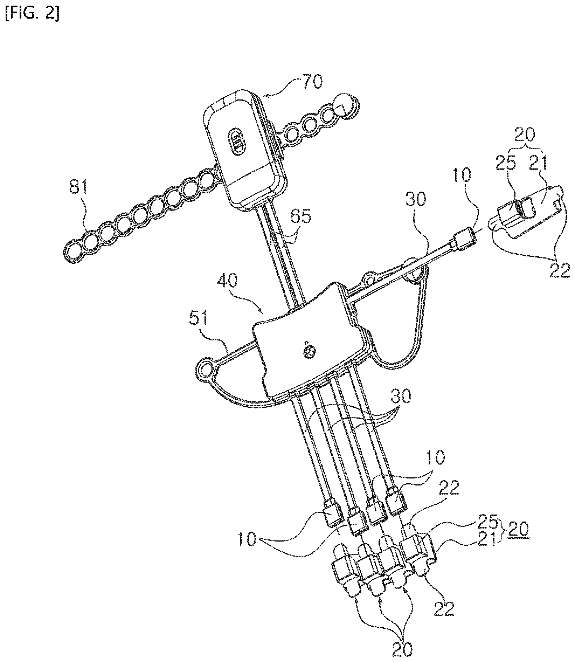

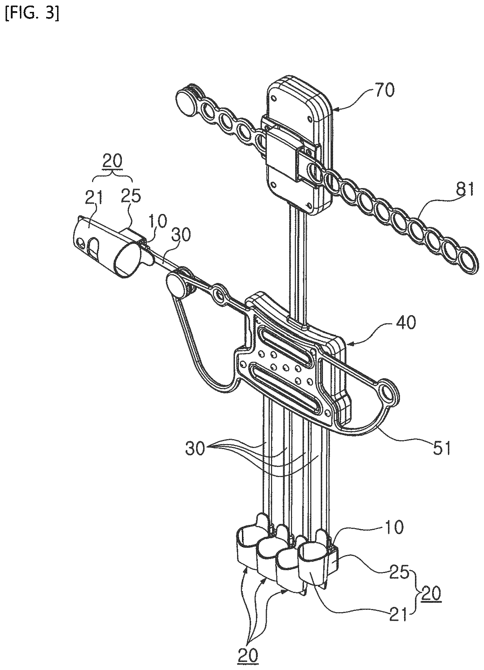

FIGS. 1 to 5 show a hand rehabilitation exercise device according to a first embodiment of the present disclosure.

As shown in these drawings, the hand rehabilitation exercise device 1a according to the first embodiment of the present disclosure includes a plurality of finger movement sensing sensors 10 and a finger worn unit 20.

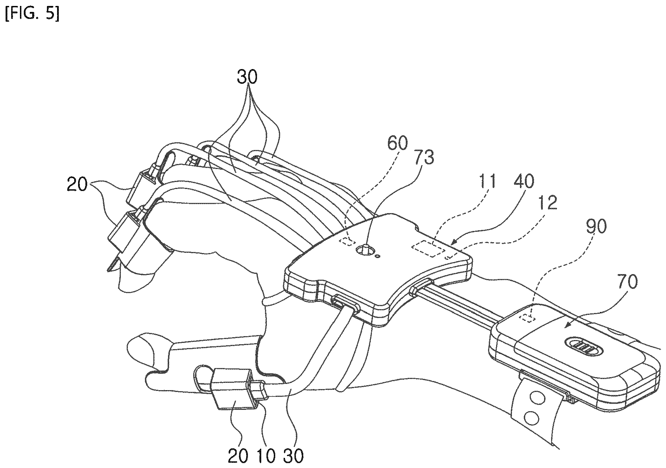

The finger movement sensing sensor 10 is disposed on the finger, preferably disposed on the finger joint, and senses the movement of the finger. As shown in FIG. 5, the finger movement sensing sensor 10 is shown as being disposed on the second finger joint from the fingertip, but the present disclosure is not limited thereto. The finger movement sensing sensor 10 may also be disposed on the remaining finger joints.

The finger movement sensing sensor 10 includes an inertial measurement unit (IMU) sensor.

The IMU sensor may be a 9-axis IMU sensor based on Microelectromechanical System (MEMS). The 9-axis IMU sensor includes a 3-axis acceleration sensor, a 3-axis gyroscope sensor, and a 3-axis terrestrial magnetism sensor. The 3-axis acceleration sensor measures the movement inertia (acceleration) of the x-axis, y-axis, and z-axis. The 3-axis gyroscope sensor measures the rotational inertia (angular velocity) of the x-axis, y-axis, and z-axis. The 3-axis terrestrial magnetism sensor measures the azimuth (direction of terrestrial magnetism) of the x-axis, y-axis, and z-axis. Here, instead of the IMU sensor as the finger movement sensing sensor 10, a flex sensor for measuring the bending displacement of the finger, an acceleration sensor, and a gyroscope sensor may be disposed.

The finger movement sensing sensor 10 is attached/detached to/from the finger worn unit 20.

The finger worn unit 20 is worn on the finger, and includes a finger tube 21 and a finger movement sensing sensor attaching/detaching unit 25.

The finger tube 21 has a hollow tube shape to fit into a finger. Each finger tube 21 has a length at which the tip of each finger may be exposed when mounted on the corresponding finger joint. The finger tube 21 may be made of an elastic material, for example, a rubber material, or made of a stretchable material so that the finger tube 21 is fitted in close contact with the finger.

A pair of wings 22 are protruded from both ends of the finger tube 21. Each wing 22 is disposed to partially wrap the upper part of the finger, but each wing 22 may be used while fitting or removing the finger tube 21 into/from the finger.

In the present embodiment, the pair of wings 22 are shown as being formed to protrude from both ends of the finger tube 21 so as to partially wrap the upper part of the finger, but the present disclosure is not limited thereto. For example, a pair of wings is formed to protrude from both ends of the finger tube so as to partially wrap the lower part of the finger, or one wing of the pair of wings is formed to protrude from one end of the finger tube so as to partially wrap the upper part of the finger, and the remaining wings may be formed to protrude from the other end of the finger tube so as to partially wrap the lower part of the finger.

In the present embodiment, the finger tube 21 is shown to be worn on the second finger joint from the fingertip, but the present disclosure is not limited thereto, and the finger tube 21 may be worn on the remaining finger joints.

The finger movement sensing sensor attaching/detaching unit 25 is provided on one side of the finger tube 21, and the finger movement sensing sensor 10 is detachably fitted.

The finger movement sensing sensor attaching/detaching unit 25 in this embodiment is formed as a rectangular receiving groove, but is not limited thereto. The finger movement sensing sensor attaching/detaching unit 25 is made of a hook shape with an upper side open, so that the finger movement sensing sensor 10 is inserted and coupled from the upper side to the lower side of the finger movement sensing sensor attaching/detaching unit 25, so that the finger movement sensing sensor attaching/detaching unit 25 may support both sides of the finger movement sensing sensor 10.

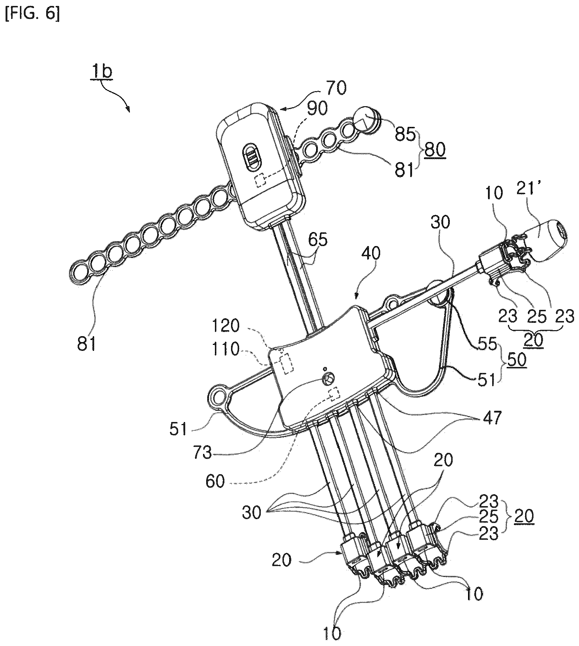

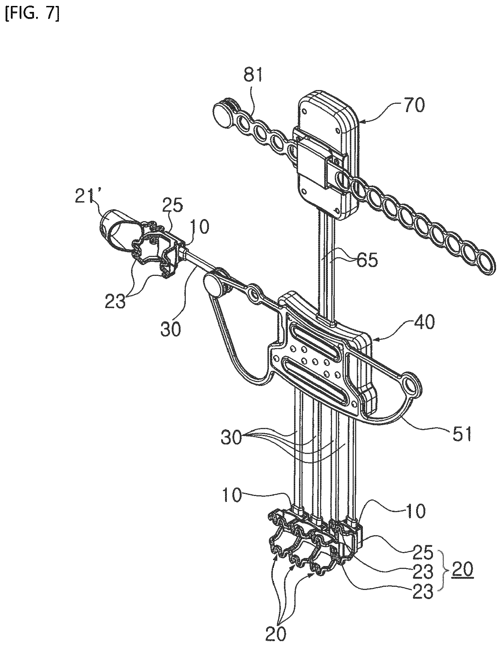

As another embodiment of the finger worn unit, as shown in FIGS. 6 and 7, the finger worn unit 20 may be formed in a ring shape. When the finger worn unit 20 has a ring shape, even when an area of the finger joint is partially contracted during the joint motion of the finger, joint motion of the finger can be naturally induced.

The finger worn unit 20 according to another embodiment includes a pair of mounting rings 23 and a finger movement sensing sensor attaching/detaching unit 25.

A pair of mounting rings 23 are spaced apart from each other, and the fingers pass through and are fitted and coupled.

The finger movement sensing sensor attaching/detaching unit 25 is connected to a pair of mounting rings 23, and the finger movement sensing sensor 10 is fitted and coupled. The finger movement sensing sensor attaching/detaching unit 25 is formed as a rectangular receiving groove so that the finger movement sensing sensor 10 is inserted and coupled.

In addition, as shown in FIG. 6, in the case of a finger tube worn on the thumb, an auxiliary finger tube 21' in the form of a thimble enclosing the tip of the thumb may be further included.

As such, the finger worn unit 20 according to another embodiment has a configuration in which a pair of mounting rings 23 are worn on the finger joints at intervals. Hence, as compared to the tube-shaped finger worn unit 20 according to the above-described embodiment, it is possible to naturally induce the joint motion of the finger even when an area of the finger joint is partially contracted during joint motion of the finger.

The plurality of finger movement sensing sensors 10 are electrically connected to the plurality of cables 30.

The cable 30 has a band shape extending in the length direction of the finger. The cable 30 is made of a stretchable material so as to be flexibly bent with respect to the joint motion of the fingers. A plurality of cables 30 interconnect each finger movement sensing sensor 10 and the back of hand worn unit 40.

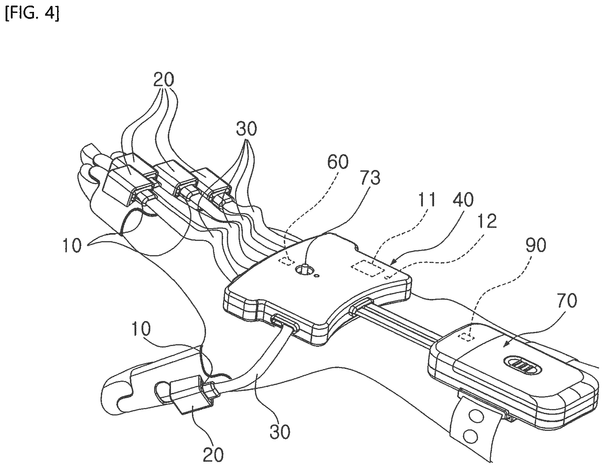

As an example, the cable 30 preferably has a length such that when the finger is bent to the maximum, the cable 30 is not bent and flatly unfolded. Accordingly, the cable 30 according to the present disclosure is curvedly contracted according to the joint motion of the finger worn on the finger worn unit 20, for example, when the finger is not bent to the maximum, by the elastic force of the cable 30, one area of the cable 30 has a shape in which the cable 30 protrudes to be partially curved as shown in FIG. 4 by the elastic force.

The cable 30 of the hand rehabilitation exercise device 1a according to the first embodiment of the present disclosure is provided in various lengths in correspondence with various finger lengths. Thus, by selecting the cable 30 in correspondence with the finger length on which the hand rehabilitation exercise device 1a according to the first embodiment of the present disclosure is worn, the device may be used by connecting the cable 30 to the finger movement sensing sensor 10 and the back of hand worn unit 40.

The cable 30 is made of an optical cable, and in addition to the function of interconnecting the finger movement sensing sensor 10 and the back of hand worn unit 40, the cable may have a function of supplying power to the finger movement sensing sensor 10, and transmitting the finger movement data sensed in the finger movement sensing sensor 10 to the control unit 110 to be described later.

The back worn unit 40 has a shape similar to the back of a human hand, and is worn on the back of hand of a rehabilitation exerciser.

A plurality of cable mounting holes 47 to which each cable 30 is mounted are formed in the back of hand worn unit 40.

In addition, the back of hand worn unit 40 is mounted on the hand by a back of hand mounting unit 50. In the present embodiment, the back of hand mounting unit 50 includes a band 51 and a locking protrusion 55.

The band 51 is disposed on both sides of the back of hand worn unit 40. The band 51 has a locking groove 53 through which the locking protrusion 55 is fitted. The locking groove 53 has a circular cross-sectional shape, but the locking groove 53 may have a cross-sectional shape such as an ellipse or a polygon, in addition to a circular shape.

Accordingly, as the locking protrusion 55 of the back of hand worn unit 40 is fitted into the locking groove 53, the back of hand worn unit 40 may be stably worn on the back of hand of a rehabilitation exerciser.

In the present embodiment, the locking groove 53 and the locking protrusion 55 are provided as the back of hand mounting unit 50, but is not limited thereto, and a Velcro cloth and double-sided tape may be provided.

In addition, the hand rehabilitation exercise device 1a according to the first embodiment of the present disclosure may further include a back movement sensing sensor 60 for sensing the movement of the back of hand.

The back of hand movement sensing sensor 60 includes an IMU sensor.

The IMU sensor may be a 9-axis IMU sensor based on Microelectromechanical System (MEMS). The 9-axis IMU sensor includes a 3-axis acceleration sensor, a 3-axis gyroscope sensor, and a 3-axis terrestrial magnetism sensor. Here, instead of the IMU sensor as the back of hand movement sensing sensor 10, a flex sensor, an acceleration sensor, and a gyroscope sensor may be disposed.

In addition, the hand rehabilitation exercise device 1a according to the first embodiment of the present disclosure may further include a wrist worn unit 70.

The wrist worn unit 70 includes a hollow cylindrical housing 71, and the housing 71 includes a finger movement sensing sensor 10, a back of hand movement sensing sensor 60, and a wrist movement sensing sensor 90 to be described later, and a battery (not shown) for supplying power to a communication module 120 to be described later are accommodated.

The housing 71 is worn on the wrist of a rehabilitation exerciser by the wrist mounting unit 80. In this embodiment, as the wrist mounting unit 80 includes a strap 81 and a locking protrusion 85.

The strap 81 has a band shape of a certain length, and the strap 81 is disposed on both sides of the housing 71 at intervals. A plurality of locking grooves 83 are formed through the strap 81 in the length direction of the strap 81. The locking groove 83 has a circular cross-sectional shape, but the locking groove 83 may have a cross-sectional shape such as an oval or a polygon in addition to a circular shape.

The locking protrusion 85 is formed to protrude from one side of the strap 81, and the locking protrusion 85 is selectively fitted into any one of the plurality of locking grooves 83 formed in the strap 81.

Accordingly, as the locking protrusion 85 of the wrist worn unit 70 is fitted into the locking groove 83, the wrist worn unit 70 may be stably worn on the wrist of a rehabilitation exerciser.

In the present embodiment, the locking groove 83 and the locking protrusion 85 are provided as the wrist mounting unit 80, but is not limited thereto, and Velcro cloth and double-sided tape may be provided.

In addition, the hand rehabilitation exercise device 1a according to the first embodiment of the present disclosure may further include a wrist movement sensing sensor 90 for sensing the movement of a wrist.

The wrist motion detection sensor 90 is accommodated in the housing 71 of the wrist worn unit 70 to sense the movement of a wrist.

The wrist movement sensing sensor 90 includes an IMU sensor.

The IMU sensor may be a 9-axis IMU sensor based on Microelectromechanical System (MEMS). The 9-axis IMU sensor includes a 3-axis acceleration sensor, a 3-axis gyroscope sensor, and a 3-axis terrestrial magnetism sensor.

Accordingly, the IMU sensor that senses the movement of a wrist may sense wrist flexion in the downward direction, wrist extension in the upward direction, wrist flexion in the left direction (Radial flexion), wrist flexion in the right direction (Ulnar flexion), and wrist rotation. Here, instead of the IMU sensor as the wrist movement sensing sensor 10, a flex sensor, an acceleration sensor, and a gyroscope sensor may be disposed.

The wrist worn unit 70 and the back of hand warning unit 40 are interconnected by a plurality of auxiliary cables 65.

Accordingly, when the finger on which the finger worn unit 20 is mounted performs joint motion in a state in which the wrist worn unit 70 is worn on the user's wrist with the strap 81, the back of hand worn unit 40 and the finger movement sensing sensor 10 do not come off the hand.

The auxiliary cable 65 is made of an optical cable, and in addition to the function of interconnecting the wrist movement sensing sensor 90 and the back of hand worn unit 40, the auxiliary cable may have a function of supplying power to the wrist movement sensing sensor 90, and transmitting the wrist movement data sensed in the wrist movement sensing sensor 90 to the control unit 110 to be described later.

The hand rehabilitation exercise device 1a according to the first embodiment of the present disclosure further includes a control unit 110.

The control unit 110 is provided on the back of hand worn unit 40.

The control unit 110 is electrically connected by each finger movement sensing sensor 10 and the cable 30, is electrically connected to the back of hand movement sensing sensor 60, is connected by the wrist movement sensing sensor 90 and the auxiliary cable 65, and receives and collects each piece of movement data of the finger, wrist, and back of hand sensed by the finger movement sensing sensor 10, the back of hand movement sensing sensor 60, and the wrist movement sensing sensor 90.

In addition, the control unit 110 transmits each piece of movement data of a finger, wrist, and hand to a server and a terminal, which are communication terminals through the communication module 120.

The communication module 120 is synchronized to enable data communication between the communication terminal and the hand rehabilitation exercise device 1a according to the first embodiment of the present disclosure. The communication module 120 provides a communication terminal with each piece of movement data of a finger, wrist, and back of hand sensed by each of the finger movement sensing sensor 10, the back of hand movement sensing sensor 60, and the wrist movement sensing sensor 90 in real time. The communication terminal receives the user's hand gesture information transmitted from the hand rehabilitation exercise device 1a according to the present disclosure through the communication module 120 in real time, for example, each piece of movement data of the finger, wrist, and back of hand, and generates a virtual hand-shaped object corresponding to the received hand gesture information of a user and displays same on a display screen. Accordingly, the user can immediately visually check the hand gesture of the user through the screen in the course of the finger rehabilitation exercise, thereby being able to be motivated for the rehabilitation exercise and to be interested in the rehabilitation exercise.

It is explained in this embodiment that the control unit 110 is communicatively connected to each of the finger movement sensing sensor 10, the back of hand movement sensing sensor 60, and the wrist movement sensing sensor 90 by cables 30 and 65, but is not limited thereto. The control unit 110 is communicatively connected to each of the finger movement sensing sensor 10, the back of hand movement sensing sensor 60, and the wrist movement sensing sensor 90 in a wireless communication method, and receives each piece of movement data of a finger, a wrist, and a back sensed by each of the finger movement sensing sensor 10, the back of hand movement sensing sensor 60, and the wrist movement sensing sensor 90. Wireless communication methods include near field communication (NFC), wireless USB, ultra wide band (UWB), Wi-Fi, Bluetooth, ZIGBEE, radio frequency (RF), and infrared data association (IrDA).

The undescribed reference numeral 73 is an operation button for operating the operation of the hand rehabilitation exercise device 1a according to the first embodiment of the present disclosure.

With this configuration, a process of hand rehabilitation training using the hand rehabilitation exercise device 1a according to the first embodiment of the present disclosure will be described.

First, the finger worn unit 20 is worn on each second joint of the thumb, index finger, middle finger, ring finger, and pinky finger.

Next, the back of hand worn unit 40 and the wrist worn unit 70 are worn on the back of hand and the wrist using the back of hand mounting unit 50 and the wrist mounting unit 80, respectively.

Next, the finger movement sensing sensor 10 connected to one end of the cable 30 is inserted and coupled to the finger movement sensing sensor attaching/detaching unit 25 of each finger worn unit 20.

Subsequently, the other end of the cable 30 is mounted on the cable mounting hole 47 of the back of hand worn unit 40, and the finger movement sensing sensor 10 is supported on the back of hand worn unit 40 through the cable 30.

Accordingly, as shown in FIG. 4, the hand rehabilitation exercise device 1a according to the first embodiment of the present disclosure is worn on the hand to be rehabilitated.

In this way, the finger worn unit 20 is first worn on the finger, and then the finger movement sensing sensor 11 is mounted on the finger movement sensing sensor attaching/detaching unit 25 of the finger worn unit 20, so that the hand rehabilitation exercise device 1a according to the first embodiment of the present disclosure can be easily worn and used even in a state in which the fingers of a patient are contracted, thereby improving user convenience. In addition, by providing the cable 30 of various lengths in correspondence with various finger lengths, the hand rehabilitation exercise device 1a according to the first embodiment of the present disclosure may select the cable 30 in correspondence with the finger lengths to be worn, the corresponding cable 30 may be connected to the finger movement sensing sensor 10 and the back of hand worn unit 40, and the device may be worn by replacing only a cable in corresponding with various finger lengths, so that the device may be used regardless of the length of fingers.

After wearing the hand rehabilitation exercise device 1a according to the first embodiment of the present disclosure, in a state in which only the back of hand and the wrist are fixed, only a specific finger is bent, or any one of the thumb and the other fingers is bent to perform a pinch operation. When all fingers are bent as shown in FIG. 5, based on the finger movement data sensed by the finger movement sensing sensor 10, it is possible to check in what form the finger exercises, for example, the degree to which the finger is bent or moved left and right through the control unit 110.

In addition, when the back of hand exercises in a state where only each finger and wrist are fixed, based on the back of hand movement data sensed by the back of hand movement sensing sensor 60, it is possible to check in what form the back of hand exercises, for example, the degree to which the back of hand is bent or twisted through the control unit 110.

In addition, when the wrist exercises in a state where each finger and the back of hand are fixed, based on the wrist movement data sensed by the wrist movement sensing sensor 90, it is possible to check in what form the wrist performs joint motion, for example, the degree to which the wrist is moved up and down or twisted through the control unit 110.

In addition, when each finger, back of hand, and wrist are exercised in combination, based on each piece of movement data of the finger, wrist, and back of hand sensed by the finger movement sensing sensor 10, the back of hand movement sensing sensor 60, and the wrist movement sensing sensor 90, it is possible to check in what form the hand exercises through the control unit 110.

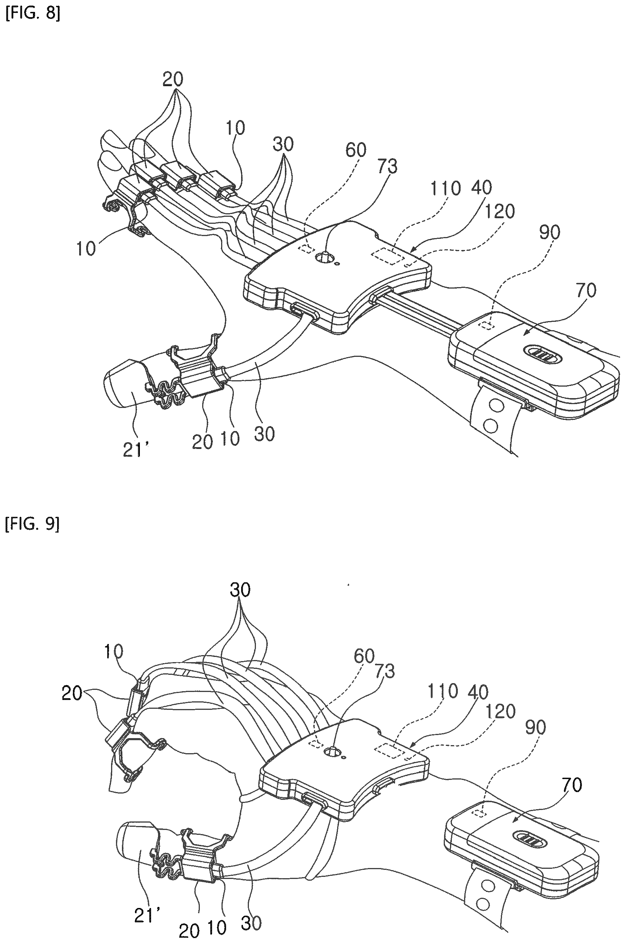

After the hand rehabilitation exercise device 1b according to the second embodiment of the present disclosure is worn on the hand to be rehabilitated as shown in FIG. 8, a specific finger or all fingers are bent as shown in FIG. 9, based on the finger movement data sensed by the finger movement sensing sensor 10, it is possible to check in what form the finger performs joint motion through the control unit 110.

As such, after the hand rehabilitation exercise device 1b according to the second embodiment of the present disclosure is worn, when the back of hand exercises in a state where only each finger and wrist are fixed, based on the back of hand movement data sensed by the back of hand movement sensing sensor 60, it is possible to check in what form the back of hand is exercised through the control unit 110.

In addition, after the hand rehabilitation exercise device 1b according to the second embodiment of the present disclosure is worn, when the wrist exercises in a state in which each finger and the back of hand are fixed, based on the wrist movement data sensed by the wrist movement sensing sensor 90, it is possible to check in what form the wrist performs joint motion through the control unit 110.

In addition, after the hand rehabilitation exercise device 1b according to the second embodiment of the present disclosure is worn, when each finger, back of hand, and wrist are exercised in combination, based on each piece of movement data of the finger, wrist, and back of hand sensed by the finger movement sensing sensor 10, the back of hand movement sensing sensor 60, and the wrist movement sensing sensor 90, it is possible to check in what form the hand exercises through the control unit 110.

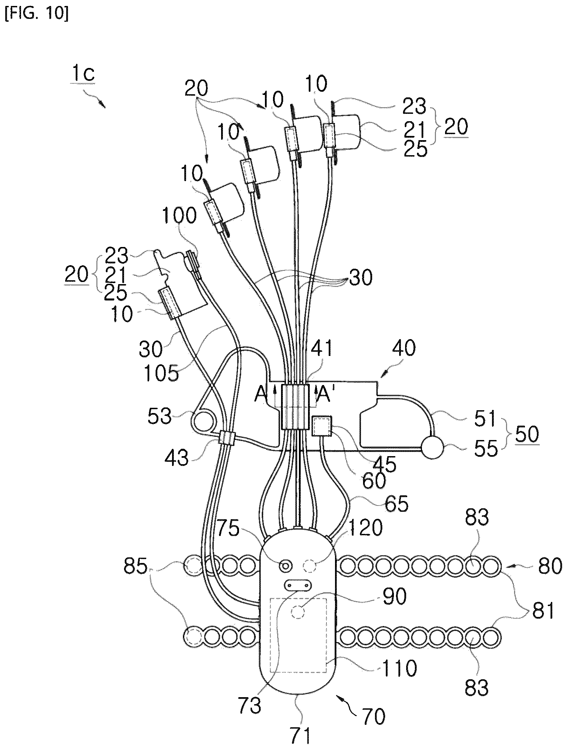

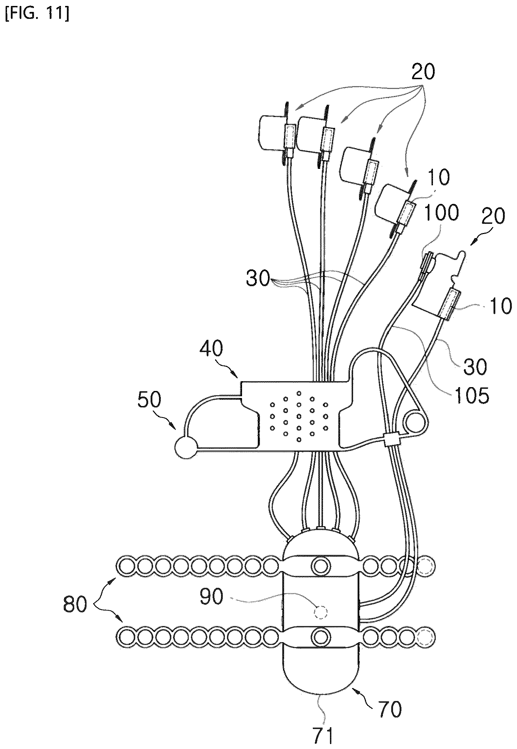

FIGS. 10 to 16 show a hand rehabilitation exercise device according to a third embodiment of the present disclosure.

In the hand rehabilitation exercise device 1c according to the third embodiment of the present disclosure, unlike the above-described embodiments, the control unit 110 is not provided on the back of hand worn unit 40, but is provided on the wrist worn unit 70.

The control unit 110 is mounted on a printed circuit board, and is accommodated in the housing 71 of the wrist worn unit 70. The control unit 110 is electrically connected to the plurality of finger movement sensing sensors 10 and the plurality of cables 30, and receives and collects finger movement data sensed by each finger movement sensing sensor 10.

In addition, the control unit 110 is electrically connected to the wrist movement sensing sensor 90 and the auxiliary cable 65, and receives and collects wrist movement data sensed by the wrist movement sensing sensor 90.

A path of a plurality of cables 30 connecting the finger movement sensing sensor 10 and the control unit 110 is formed in the back of hand worn unit 40.

Specifically, on one side of the back of hand worn unit 40, a cable aligning unit 41 in which a plurality of cables 30 are fixedly or movably aligned is provided, and a path of the plurality of cables 30 is formed in the back of hand worn unit 40.

The cable aligning unit 41 has a shape in which a plurality of tubes are disposed side by side so that the cables 30 corresponding to the remaining fingers other than the thumb are aligned. The cable aligning unit 41 is disposed on the back of hand worn unit 40 to be biased on one side of the back of hand worn unit 40, for example, adjacent to the thumb.

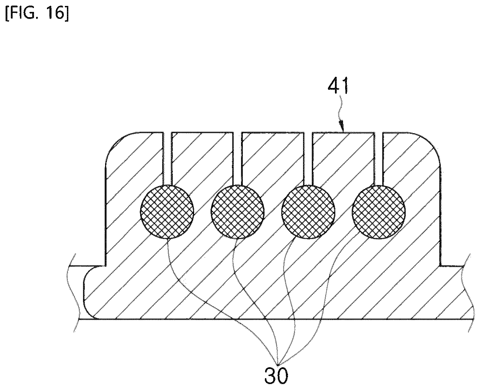

As shown in-FIG. 16, the cable aligning unit 41 has a plurality of protrusion shapes that accommodate and partially surround each cable 30, and thus each cable 30 may be detachably installed from the cable aligning unit 41.

Accordingly, by detachably installing each cable 30 to the cable aligning unit 41, the length of each cable 30 may be adjusted in response to the distance between the finger movement sensing sensor 10 and the cable aligning unit 41 disposed in each finger joint.

In addition, as the length between the finger movement sensing sensor 10 and the cable aligning unit 41 respectively disposed on the ring finger and the pinky finger increases, the phenomenon of interference with the cable 30 disposed on the index finger and middle finger may be prevented.

In particular, even when the finger length is different for each wearer, the length of the cable 30 may be provided according to the length of the wearer's finger by the cable aligning unit 41.

In the present embodiment, the remaining cables 30 other than the thumb and index finger among the plurality of cables 30 connected to each finger movement sensing sensor 10 are not disposed side by side along the length direction of each finger, and are disposed and biased near the thumb by the cable aligning unit 41. Accordingly, in the case of a rehabilitation exerciser having a contracted finger, the cable 30 becomes bendable in response to the state of the finger and its movement, thereby eliminating the discomfort of the cable 30 sliding on the back of hand during joint motion of the finger and enabling rehabilitation exercises.

In addition, the thumb cable 30 is provided on one side of the band 51 of the back of hand worn unit 40 together with a pressure measuring sensor cable 105 to be described later. The cable 30 for the thumb is fixedly or movably aligned to the thumb cable aligning unit 43.

The plurality of cables 30 may be formed of a wire having a flexibly bendable circular cross-section.

In addition, on the other side of the back of hand worn unit 40, for example, in the central area of the back of hand, there is provided a back of hand movement sensing sensor attaching/detaching unit 45 to which the back of hand movement sensing sensor 60 is attachable and detachable.

As such, as the back of hand movement sensing sensor 60 is disposed in the central area of the back of hand worn unit 40, it is possible to improve the degree of measurement of the movement of the back of hand to be rehabilitated during the joint motion of the finger.

The back of hand movement sensing sensor attaching/detaching unit 45 is formed as a rectangular receiving groove so that the back of hand movement sensing sensor 60 is inserted and coupled.

The back of hand movement sensing sensor attaching/detaching unit 45 according to this embodiment is formed as a rectangular receiving groove, but is not limited thereto. The back of hand movement sensing sensor attaching/detaching unit is not shown, but is made of a hook shape with an upper side open, so that the finger movement sensing sensor is inserted and coupled from the upper side to the lower side of the finger movement sensing sensor attaching/detaching unit, so that the finger movement sensing sensor attaching/detaching unit may support both sides of the finger movement sensing sensor.

In addition, the back of hand movement sensing sensor 60 is detachably fitted and coupled to the back of hand movement sensing sensor attaching/detaching unit 45 of the back of hand worn unit 40.

The back of hand movement sensing sensor 60 is electrically connected to the control unit 110 by an auxiliary cable 65. The back of hand movement data sensed by the back of hand movement sensing sensor 60 is transmitted to the control unit 110 through the auxiliary cable 65.

In addition, on the housing 71 of the wrist worn unit 70, an operation button 73 for turning on or off the operation of the hand rehabilitation exercise device 1c according to the third embodiment of the present disclosure, and a display lamp 75 indicating an operation state are provided.

In addition, the hand rehabilitation exercise devicelc according to the third embodiment of the present disclosure may further include a pressure measuring sensor 100.

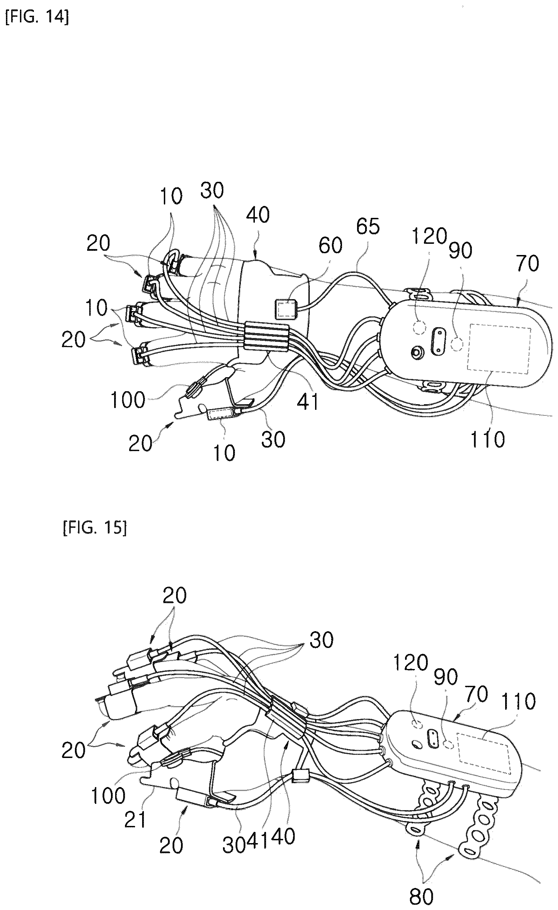

FIG. 15 is a view illustrating a state of pinching the thumb and index finger in a state in which the hand rehabilitation exercise device 1c according to the third embodiment of the present disclosure is worn.

The pressure measuring sensor 100 may have a load cell shape, and is provided in the finger worn unit 20 worn on the thumb.

Accordingly, the pressure measuring sensor 100 contacts any one of the index finger, middle finger, ring finger, and pinky finger to measure the pressure with the thumb, for example, the pinch force with the thumb.

The pressure measuring sensor 100 is electrically connected to the control unit 110 provided in the wrist worn unit 70 by the pressure measuring sensor cable 105.

Accordingly, the control unit 110 of the hand rehabilitation exercise device 1c according to the third embodiment of the present disclosure is electrically connected by the pressure measuring sensor 100 and the pressure measuring sensor cable 105, and receives and collects the pressure data measured in the pressure measuring sensor 100.

In addition, the control unit 110 may transmit each piece of movement data of a finger, a wrist, and a back of hand, and pressure data to a server and a terminal, which are communication terminals (not shown) through the communication module 120.

The communication module 120 may be provided in the housing 71 of the wrist worn unit 70.

The communication module 120 is synchronized to enable data communication between the communication terminal and the hand rehabilitation exercise device 1 according to an embodiment of the present disclosure. The communication module 120 provides a communication terminal with each piece of movement data of a finger, wrist, and back of hand sensed by each of the finger movement sensing sensor 10, the back of hand movement sensing sensor 60, and the wrist movement sensing sensor 90, and the pressure data measured by the pressure measuring sensor 100 in real time.

The communication terminal receives the hand gesture information of a rehabilitation exerciser transmitted from the hand rehabilitation exercise device 1 according to the present disclosure through the communication module 120 in real time, for example, each piece of movement data of the finger, wrist, and back of hand and the pressure data measured by the pressure measuring sensor 100, and generates a virtual hand-shaped object corresponding to the received hand gesture information of the rehabilitation exerciser and displays same on a display screen.

Accordingly, the rehabilitation exerciser can immediately visually check the hand gesture of the rehabilitation exerciser through the screen in the course of the finger rehabilitation exercise, thereby being able to be motivated for the rehabilitation exercise and to be interested in the rehabilitation exercise.

It is explained in this embodiment that the control unit 110 is communicatively connected to each of the finger movement sensing sensor 10, the back of hand movement sensing sensor 60, the wrist movement sensing sensor 90, and the pressure measuring sensor 100 by cables 30, 65 and 105, but is not limited thereto. The control unit 110 is communicatively connected to each of the finger movement sensing sensor 10, the back of hand movement sensing sensor 60, the wrist movement sensing sensor 90, and the pressure measuring sensor 100 in a wireless communication method, and receives each piece of movement data and pressure data of a finger, a wrist, and a back sensed by each of the finger movement sensing sensor 10, the back of hand movement sensing sensor 60, and wrist movement sensing sensor 90, and the pressure measuring sensor 100. Wireless communication methods include near field communication (NFC), wireless USB, ultra wide band (UWB), Wi-Fi, Bluetooth, ZIGBEE, radio frequency (RF), and infrared data association (IrDA).

With this configuration, a process of wearing the hand rehabilitation exercise device 1c according to the third embodiment of the present disclosure will be described.

First, the finger worn unit 20 is worn on the first joint of the thumb and the second joint of each of the index finger, middle finger, ring finger, and pinky finger.

Next, the back of hand worn unit 40 and the wrist worn unit 70 are worn on the back of hand and the wrist using the back of hand worn unit 50 and the wrist worn unit 80, respectively.

Subsequently, the cable 30 supported by the wrist worn unit 70 is disposed toward each finger worn unit 20 through the cable aligning unit 41 and the thumb cable aligning unit 43 of the back of hand worn unit 40.

Subsequently, the finger movement sensing sensor 10 connected to one end of each cable 30 is inserted and coupled to the finger movement sensing sensor attaching/detaching unit 25 of each finger worn unit 20.

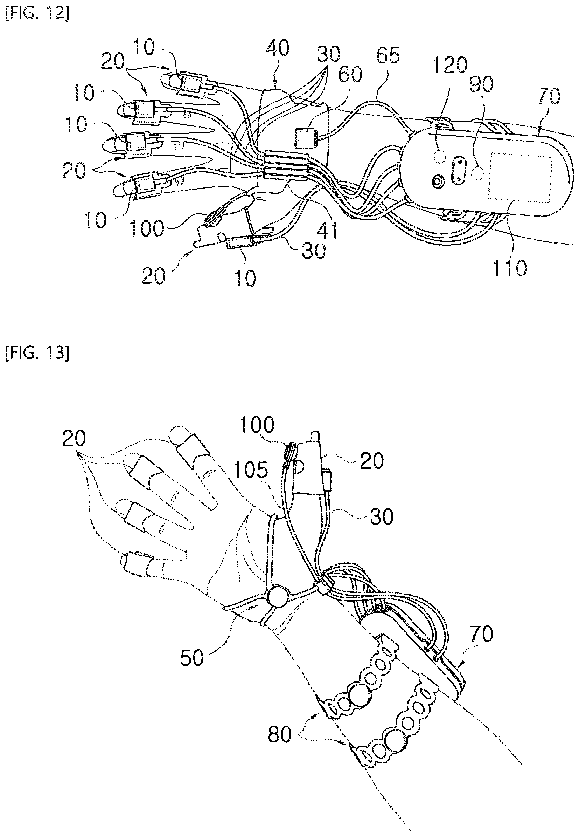

Accordingly, the hand rehabilitation exercise device 1c according to the third embodiment of the present disclosure is mounted on the hand to be rehabilitated as shown in FIGS. 12 and 13.

In this way, each finger worn unit 20 is first worn on each finger, and then the finger movement sensing sensor 11 is mounted on the finger movement sensing sensor attaching/detaching unit 25 of the finger worn unit 20, so that the hand rehabilitation exercise device 1a according to the third embodiment of the present disclosure can be easily worn and used even in a state in which the fingers of an rehabilitation exerciser are contracted, thereby improving convenience of the rehabilitation exerciser.

In addition, by providing the cable 30 of various lengths in correspondence with various finger states, the hand rehabilitation exercise device 1c according to the third embodiment of the present disclosure may select the cable 30 in correspondence with the finger states to be worn, the corresponding cable 30 may be connected to the finger movement sensing sensor 10 and the back of hand worn unit 70, and the device may be worn by replacing only the cable 30 in corresponding with various finger states, so that the device may be used regardless of the state of fingers.

Accordingly, when the rehabilitation exerciser jointly moves the fingers as shown in FIG. 14 while wearing the hand rehabilitation exercise device 1c according to the third embodiment of the present disclosure, the rehabilitation exerciser may perform rehabilitation exercises by checking the degree of bending of each finger through the control unit 110 based on the finger movement data sensed by each finger movement sensing sensor 10.

In addition, as shown in FIG. 15, by contacting or separating the index finger toward the pressure measuring sensor 100 provided in the finger worn unit 20 worn on the thumb, the thumb and the index finger may perform pinch exercises.

When the index finger contacts or separates the pressure measuring sensor 100, based on the finger movement data sensed by the respective finger movement sensing sensor 10 of the thumb and index finger, it is possible to check, for example, the degree of bending of the finger through the controller 110 in which form the thumb and index finger perform pinch exercises.

For example, it is possible to check the pinch motion state, such as whether the index finger and thumb are in normal contact or whether the index finger and thumb are in side contact, based on the finger movement data sensed by each finger movement sensing sensor 10. Simultaneously, when the index finger contacts the pressure measuring sensor 100, the pressure measuring sensor 100 measures the pinch pressure with the thumb and index finger. Based on the pressure data measured by the pressure measuring sensor 100, it is possible to check through the control unit 110 by how much force the thumb and forefinger perform pinch exercises, for example, how much pinch force is generated by the thumb and index finger.

Accordingly, the hand rehabilitation exercise device 1c according to the third embodiment of the present disclosure may perform a rehabilitation exercise while simultaneously measuring a degree of bending of a finger and a pressure applied to the finger when the finger performs pinch exercises.

In addition, in the hand rehabilitation exercise device 1c according to the third embodiment of the present disclosure, when the back of hand is exercised in a state where only each finger and wrist are fixed, based on the back of hand movement data sensed by the back of hand movement sensing sensor 60, it is possible to check in what form the back of hand exercises, for example, the degree to which the back of hand is bent or twisted through the control unit 110.

In addition, when the wrist exercises in a state where each finger and the back of hand are fixed, based on the wrist movement data sensed by the wrist movement sensing sensor 90, it is possible to check in what form the wrist performs joint motion, for example, the degree to which the wrist is moved up and down or twisted through the control unit 110.

In addition, when each finger, back of hand, and wrist are exercised in combination, based on each piece of movement data of the finger, wrist, and back of hand sensed by the finger movement sensing sensor 10, the back of hand movement sensing sensor 60, and the wrist movement sensing sensor 90, it is possible to check in what form the hand exercises through the control unit 110.

In this way, the hand rehabilitation exercise device 1c according to the third embodiment of the present disclosure improves the wearing sensation by disposing a control unit mounted on a printed circuit board on a wrist, and enables a rehabilitation exerciser to perform rehabilitation exercises without feeling discomfort when the rehabilitation exerciser moves his/her back of the hand. In addition, the hand rehabilitation exercise device makes it possible to reduce friction between a cable and a finger during rehabilitation exercise and enables smooth rehabilitation exercises without interfering with the movement of the finger. In addition, the hand rehabilitation exercise device can be easily worn even in a state in which the fingers are contracted, thereby enabling a rehabilitation exercise.

In the above-described embodiments, when the finger movement sensing sensor, the back of hand movement sensing sensor, and the wrist movement sensing sensor are configured as an IMU sensor, the initialization process of each movement sensing sensor is not always necessary before the rehabilitation exercise, so that the convenience of the rehabilitation exerciser is improved.

The hand rehabilitation exercise device according to the present disclosure is shown as being provided with a finger movement sensing sensor and a cable to correspond to each of the thumb, index finger, middle finger, ring finger, and pinky finger, but is not limited thereto. In another embodiment, the finger movement sensing sensor and the cable may be provided to correspond to one or more of thumb, index finger, middle finger, ring finger, and pinky finger.

In addition, although the hand rehabilitation exercise device in the above-described embodiments is shown as being provided with a finger worn unit, a back of hand worn unit, and a wrist worn unit, it is not limited thereto. The hand rehabilitation exercise device may include a finger worn unit and a back of hand worn unit, or may include a finger worn unit and a wrist worn unit.

When the hand rehabilitation exercise device includes a finger worn unit and a back of hand worn unit, the finger movement sensing sensor and the back of hand worn unit may be communicatively connected by wire (cable) or wirelessly. In addition, the control unit may be provided on the back of hand worn unit to receive and collect each piece of movement data of the finger and the back of hand sensed by the finger movement sensing sensor and the back of hand movement sensing sensor. Accordingly, after the finger worn unit is worn on a specific finger joint and the back of hand worn unit is worn on the back of hand, the finger movement sensing sensor is mounted on the finger movement sensing sensor attaching/detaching unit to perform rehabilitation exercise.

When the hand rehabilitation exercise device includes a finger worn unit and a wrist worn unit, the finger movement sensing sensor and the wrist worn unit may be communicatively connected by wire (cable) or wirelessly. In addition, the control unit may be provided in the wrist worn unit to receive and collect each piece of movement data of the finger and wrist sensed by the finger movement sensing sensor and the wrist movement sensing sensor. Accordingly, after the finger worn unit is worn on a specific finger joint and the wrist worn unit is worn on a wrist, the finger movement sensing sensor is mounted on the finger movement sensing sensor attaching/detaching unit to perform rehabilitation exercise.

## Claims

1. A hand rehabilitation exercise device, comprising:
a finger movement sensing sensor disposed on a finger and senses a movement of the finger;
a back of hand worn unit communicatively connected to the finger movement sensing sensor in a wired or wireless manner and worn on the back of a hand; and
a finger worn unit having a finger movement sensing sensor attaching/detaching unit to/from which the finger movement sensing sensor is attached/detached, and which is worn on the finger,
wherein the finger worn unit is worn on a specific finger joint, the back of hand worn unit is worn on the back of a hand, and then the finger movement sensing sensor is mounted on the finger movement sensing sensor attaching/detaching unit.

2. A hand rehabilitation exercise device, comprising:
a finger movement sensing sensor disposed on a finger and senses a movement of the finger;
a wrist worn unit communicatively connected to the finger movement sensing sensor in a wired or wireless manner and worn on a wrist; and
a finger worn unit having a finger movement sensing sensor attaching/detaching unit to/from which the finger movement sensing sensor is attached/detached, and which is worn on the finger,
wherein the finger worn unit is worn on a specific finger joint, the wrist worn unit is worn on a wrist, and then the finger movement sensing sensor is mounted on the finger movement sensing sensor attaching/detaching unit.

3. The hand rehabilitation exercise device of claim 1 or 2, wherein the finger worn unit comprises:
a finger tube coupled to a finger joint; and
a finger movement sensing sensor attaching/detaching unit provided on one side of the finger tube to/from which the finger movement sensing sensor is attached/detached.

4. The hand rehabilitation exercise device of claim 1 or 2, wherein the finger worn unit comprises:
a pair of mounting rings which forms a finger mounting hole on which a finger is mounted and is disposed at a distance from each other; and
a finger movement sensing sensor attaching/detaching unit connected to the pair of mounting rings and to/from which the finger movement sensing sensor is attached/detached.

5. The hand rehabilitation exercise device of claim 1, wherein:
the finger movement sensing sensor and the back of hand worn unit are electrically connected by a cable extending in a length direction of the finger; and
the cable is curvedly stretched and contracted according to joint motion of a finger worn on the finger worn unit.

6. The hand rehabilitation exercise device of claim 1, further comprising: a back of hand movement sensing sensor disposed on the back of hand worn unit and senses a movement of a back of hand.

7. The hand rehabilitation exercise device of claim 6, further comprising: a control unit provided in the back of hand worn unit to receive and collect each piece of movement data of a finger and a back of hand sensed by the finger movement sensing sensor and the back of hand movement sensing sensor.

8. The hand rehabilitation exercise device of claim 2, wherein:
the finger movement sensing sensor and the wrist worn unit are electrically connected by a cable extending in a length direction of the finger; and
the cable is curvedly stretched and contracted according to joint motion of a finger worn on the finger worn unit.

9. The hand rehabilitation exercise device of claim 8, further comprising: a wrist movement sensing sensor wrist worn unit disposed on the wrist worn unit and sensing a movement of a wrist.

10. The hand rehabilitation exercise device of claim 9, further comprising: a control unit provided in the wrist worn unit to receive and collect each piece of movement data of a finger and a wrist sensed by the finger movement sensing sensor and the wrist movement sensing sensor.

11. The hand rehabilitation exercise device of claim 1, further comprising:
a wrist worn unit worn on a wrist; and
a wrist movement sensing sensor disposed on the wrist worn unit and sensing a movement of a wrist.

12. The hand rehabilitation exercise device of claim 11, wherein the back of hand worn unit and the wrist worn unit are communicatively connected in a wired or wireless manner.

13. The hand rehabilitation exercise device of claim 11, further comprising: a control unit provided in the back of hand worn unit or the wrist worn unit to receive and collect each piece of movement data of a finger, back of hand, and wrist sensed by the finger movement sensing sensor, the back of hand movement sensing sensor, and the wrist movement sensing sensor.

14. A hand rehabilitation exercise device, comprising:
a plurality of finger movement sensing sensors which are disposed on each of a plurality of fingers and sense a movement of each finger;
a plurality of cables respectively connected to the plurality of finger movement sensing sensors and extended by a length toward a wrist;
a back of hand worn unit that forms a path for the plurality of cables and is worn on the back of a hand;
a wrist worn unit worn on the wrist to which the plurality of cables are connected; and
a control unit mounted on a printed circuit board and provided in the wrist worn unit to receive and collect finger movement data sensed by the plurality of finger movement sensing sensors through each of the plurality of cables.

15. The hand rehabilitation exercise device of claim 14, further comprising:
a back of hand movement sensing sensor provided in the back of hand worn unit to sense a movement of a back of hand; and
an auxiliary cable electrically connecting the control unit and the back of hand movement sensing sensor,
wherein the control unit receives and collects back of hand movement data sensed by the back of hand movement sensing sensor through the auxiliary cable.

16. The hand rehabilitation exercise device of claim 15, wherein the back of hand worn unit further comprises a back of hand movement sensing sensor attaching/detaching unit to/from which the back of hand movement sensing sensor is attached/detached.

17. The hand rehabilitation exercise device of claim 16, further comprising: a cable aligning unit provided in the back of hand worn unit, and fixes or movably aligns the plurality of cables.

18. The hand rehabilitation exercise device of claim 17, wherein:
the cable aligning unit is disposed on a back of hand adjacent to the thumb; and
the back of hand movement sensing sensor attaching/detaching unit is disposed at a central area of the back of hand.

19. The hand rehabilitation exercise device of claim 14, further comprising: a wrist movement sensing sensor provided in the wrist worn unit and senses a movement of a wrist, wherein the control unit receives and collects wrist movement data sensed by the wrist movement sensing sensor.

20. The hand rehabilitation exercise device of claim 14, further comprising: a finger worn unit having a finger movement sensing sensor attaching/detaching unit to/from which the finger movement sensing sensor is attached/detached, and which is worn on the finger.

21. The hand rehabilitation exercise device of claim 14, wherein the plurality of cables is made of a bendable circular cross-section wire, and have a length corresponding to joint motion of each finger according to a state of each finger.

22. The hand rehabilitation exercise device of claim 14, further comprising: a pressure measuring sensor provided in the finger worn unit worn on the thumb, and measures pressure generated on a finger in which any one of an index finger, middle finger, ring finger, and pinky finger is in contact,
wherein the control unit receives and collects pressure data measured by the pressure measuring sensor.
